# EUROPEAN PATENT SPECIFICATION

(11) **EP 4 287 190 B1**
(45) Date of publication and mention of the grant of the patent: **08.07.2026**
(21) Application number: 23203162.5
(22) Date of filing: 17.09.2021
(51) Int. Cl.: G16B 20/20, G16B 40/20, G16B 30/10, C12Q 1/6886, C12Q 1/6869

(54) **METHOD AND APPARATUS FOR MACHINE LEARNING BASED IDENTIFICATION OF STRUCTURAL VARIANTS IN CANCER GENOMES**
VERFAHREN UND GERÄT FÜR DIE AUF MASCHINELLEM LERNEN BASIERENDE IDENTIFIZIERUNG VON STRUKTURVARIANTEN IN KREBSGENOMEN
MÉTHODE ET APPAREIL POUR L'IDENTIFICATION DE VARIANTS STRUCTURELS DANS LES GÉNOMES DU CANCER BASÉE SUR L'APPRENTISSAGE AUTOMATIQUE

(30) Priority: 17.09.2020 KR 20200119798; 27.04.2021 KR 20210054190; 15.09.2021 KR 20210123291
(43) Date of publication of application: 06.12.2023
(62) Divisional of application: 21197318.5
(73) Proprietor: Inocras Korea Inc., Daejeon (KR)
(72) Inventor: Ju, Youngseok, Daejeon (KR); Park, Hansol, Daejeon (KR); Park, Seongyeol, Daejeon (KR)
(74) Representative: GLAWE DELFS MOLL

(56) References cited:
- WO-A1-2020/035446
- US-A1- 2019 189 242
- KUHN MATTHIAS ET AL: "Finding small somatic structural variants in exome sequencing data: a machine learning approach", COMPUTATIONAL STATISTICS, PHYSICA VERLAG, DE, vol. 33, no. 3, 10 August 2016 (2016-08-10), pages 1145 - 1158, XP036537640, ISSN: 0943-4062, [retrieved on 20160810], DOI: 10.1007/S00180-016-0674-2
- ZEV N. KRONENBERG ET AL: "Wham: Identifying Structural Variants of Biological Consequence", PLOS COMPUTATIONAL BIOLOGY, vol. 7, no. 12, 1 December 2015 (2015-12-01), pages e1002216, XP055334268, DOI: 10.1371/journal.pcbi.1004572

## Description

### Technical Field

The present invention relates to the identification of structural variants in genomes.

### Background

Next generation sequencing is a high-throughput genome sequencing technology that reads the genome by fragments and analyzes the sequence of the genome by assembling the fragments. With the advent of next-generation sequencing technology, whole-genome sequencing (WGS) has been widely used for detecting almost all types of somatic mutations. Thanks to its usefulness, WGS analysis is being performed extensively and plays a critical role especially in cancer genomics.

Structural variants (SVs) play an important role in tumorigenesis, so many bioinformatics algorithms and tools have been developed to detect the somatic variants in cancer genomes. However, SV search tools inevitably include a significant number of false positives in their output to achieve high sensitivity. Therefore, the operation of generating an accurate list of SVs (i.e., true positives) by removing the false positives from among the structural variant candidates called by the structural variant search tool should be followed.

However, in order to eliminate the false positives, experts have to set the optimal filtering conditions through time-consuming and labor-intensive heuristic trial and error. As such false-positive removal work depends on human competence, there is a problem in that inter-individual and inter-laboratory variability occurs, and there is no standardized filtering rule that can be applied to various cancer genome studies, making it difficult to maintain the quality of bioinformatics interpretation and falling scalability. Additionally, from the fact that it took years to freeze the final variant call set by top-notch experts to annotate 2,658 cancer whole-genome sequences in the Pan-Cancer Analysis of Whole Genomes (PCAWG) consortium, it can be seen that the analysis takes a considerable amount of time. In particular, considering the production cost of genome data, there is a practical limit in finding structural variants using existing methods in large-scale cancer genome research or clinical environments.
Document US 2019/189242 A1 discloses a machine learning system and method for somatic mutation discovery. Inputs to a classification model include output from sequencing, analysis, and variant calling, such as the FASTQ quality score for any given base in the sequence data. By using the trained machine learning algorithm, the computer can analyze sequence reads from a target nucleic acid and reports if the nucleic acid has a sequence mutation or structural alteration.
Document WO 2020/035446 A1 discloses a method that utilizes neural networks to analyze genomic sequence data generated by a sequencer. The method can include obtaining augmented sequence reads by inserting one or more spaces in one or more sequence reads. At the output of the trained neural network, prediction data related to a variant within the plurality of sequence reads is obtained.
The publication "Finding small somatic structural variants in exome sequencing data: a machine learning approach", Kuhn Matthias et al, Computational Statistics Vol. 33, No. 3, DOI: 10.1007/S00180-016-0674-2, 2016-08-10, discloses an algorithm to call small somatic structural variants using next-generation sequencing data from both tumor and normal samples of a patient. The method comprises a screening step which selects genomic loci where the tumor sample shows an enrichment of clipped bases from either side of its mapped reads. In a second step a classifier is built to distinguish genomic regions that harbour a somatic structural variant from those regions that are essentially unchanged between normal and tumor samples.
The publication "Wham: Identifying Structural Variants of Biological Consequence", Zev N. Kronenberg et al, PLOS Computational Biology, vol. 7, no. 12, 2015-12-01, page e1002216, XP055334268, discloses a method for identifying structural variants in short-read sequence data and associating the structural variants with corresponding phenotypes. To this end, a computational tool is described which identifies structural variants in next-generation sequencing data using multiple mapping-based signals such as mate-pair mapping, split-read mapping, and soft-clipping.

### Disclosure

### Technical Problem

The present disclosure provides a method and apparatus for identifying somatic structural variants for detecting various structural variants in genome data through a machine learning model.

The present disclosure provides a method and apparatus for extracting features of structural variant candidates searched by a structural variant search tool, and identifying positive structural variants through a machine learning model that learns the features of positive structural variants (true positives) and negative structural variants (false positives).

According to an embodiment, it is possible to accurately detect various structural variants in genomes through a machine learning model, and to generate an accurate list of structural variants by removing false positives, thereby contributing to the development of oncology including cancer diagnosis.

According to an embodiment, it is possible to quickly remove false positives from structural variant candidates by using a machine learning model trained with the standardized features of structural variant candidates.

According to the embodiment, when a machine learning model trained with standardized features is used, the time required for filtering false positives by experts setting the optimal filtering conditions through heuristic trial and error through manual inspection can be significantly reduced.

According to the embodiment, scalability can be increased through standardized identification of structural variants using a machine learning model, and manual curation by experts is unnecessary, thereby reducing cost and time.

According to an embodiment, it can be applied to various cohorts through simple re-training of the machine learning model, and an optimal cutoff can be found based on classification performance.

### Technical Solution

According to the present disclosure, a method performed by a computing device for identifying structural variants in genomes is provided. The method may comprise obtaining structural variant candidates identified from whole-genome sequencing data including a pair of tumor genome data and normal tissue genome data, extracting features of each structural variant candidate based on data located within a predetermined range from each structural variant candidate in the whole-genome sequencing data, labeling each structural variant candidate with classification information based on a list of known structural variants, and training a machine learning model by using a dataset, in which features of each structural variant candidate and the labeled classification information are annotated, wherein the machine learning model receives an identification target structural variant candidate and outputs a classification of the identification target structural variant candidate.

According to an embodiment, the method may further comprise, modifying an estimated position of a first structural variant candidate based on supplementary alignment (SA) tag information associated with the first structural variant candidate among the structural variant candidates before extracting features of each structural variant candidate.

According to an embodiment, modifying the estimated position of the first structural variant candidate may comprise identifying a first region and a second region on a reference sequence, to which the first structural variant candidate is mapped, and the first region and the second region may be regions that are not adjacent to each other.

According to an embodiment, modifying the estimated position of the first structural variant candidate may comprise determining a position of a breakpoint associated with the first structural variant candidate.

According to an embodiment, extracting features of each structural variant candidate based on data located within a predetermined range from each structural variant candidate may comprise, obtaining data of a first length or more in a direction, in which variant-supporting reads of the first structural variant candidate are located, from a breakpoint associated with the first structural variant candidate among the structural variant candidates in the whole-genome sequencing data.

According to an embodiment, the first length distance may be an average insert size of the whole-genome sequencing data.

According to an embodiment, the features of the structural variant candidate may include at least one of a number of variant-supporting reads, a number of split reads, a number of split reads with supplementary alignment (SA) tags, and a number of reads having the same clipped sequences as split reads within normal tissue genome data.

According to an embodiment, extracting features of each structural variant candidate based on data located within a predetermined range from each structural variant candidate may comprise obtaining data of a second length or less in a direction opposite to variant-supporting reads of a first structural variant candidate from a breakpoint associated with the first structural variant candidate among the structural variant candidates in the whole-genome sequencing data.

According to an embodiment, the second length may be 200 base pairs.

According to an embodiment, labeling with the classification information may comprise, labeling a first structural variant candidate among the structural variant candidates as positive, wherein a position of the first structural variant candidate is marked as positive in the list of known structural variants and labeling a second structural variant candidate among the structural variant candidates as negative, wherein a position of the second structural variant candidate is marked as negative in the list of known structural variants.

According to an embodiment, extracting the features of each structural variant candidate may comprise, extracting, for each structural variant candidate, a number of variant-supporting reads, a number of split reads with supplementary alignment tag, a number of split reads, mapping quality, read depth change, a number of background noise reads, and a number of samples, in which the same variant is detected among the panel of normal tissue genome data.

According to an embodiment, extracting the features of each structural variant candidate may comprise, extracting, for each structural variant candidate, tumor histology, whole-genome duplication status, tumor purity, and tumor ploidy of a tumor genome from clinical data.

According to an embodiment, the machine learning model may receive features of the identification target structural variant candidate and outputs a probability value for classifying the identification target structural variant candidate as positive or negative.

According to an embodiment, the method may further comprise, evaluating classification performance of the machine learning model by using the validation samples included in the whole-genome sequencing data, and determining a cutoff value for determining whether a probability value output from the machine learning model indicates positive or negative based on the classification performance validation result of the machine learning model.

According to an embodiment, obtaining structural variant candidates identified from the whole-genome sequencing data may comprise, inputting the whole-genome sequencing data into a structural variant search tool; and obtaining structural variant candidates output by the structural variant search tool.

According to the present disclosure, a method performed by a computing device for identifying structural variants in genomes is provided. The method may comprise obtaining structural variant candidates from identification target whole-genome sequencing data, extracting features of each structural variant candidate based on data located within a predetermined range from each structural variant candidate in the whole-genome sequencing data, and inputting the extracted features of each structural variant candidate into a trained machine learning model to identify each structural variant candidate as a negative structural variant candidate or a positive structural variant candidate, wherein the machine learning model is trained by using features of structural variant candidates for training obtained from whole-genome sequencing data including a pair of tumor genome data and normal tissue genome data, and classification information of the structural variant candidate for training.

According to an embodiment, the method may further comprise generating a list of true structural variants by removing structural variant candidates identified as the negative structural variant from among the obtained structural variant candidates.

According to the present disclosure, a computer readable non-transitory storage medium is provided. The storage medium may include instructions that, when executed by one or more processors of a computing device, cause the computing device to perform operations comprising obtaining structural variant candidates identified from whole-genome sequencing data including a pair of tumor genome data and normal tissue genome data, extracting features of each structural variant candidate based on data located within a predetermined range from each structural variant candidate in the whole-genome sequencing data, labeling each structural variant candidate with classification information based on a list of known structural variants, and training a machine learning model by using a dataset, in which features of each structural variant candidate and the labeled classification information are annotated, wherein the machine learning model receives an identification target structural variant candidate and outputs a classification of the identification target structural variant candidate.

According to the present disclosure, a computing device is provided. The computing device may comprise a processor, and a memory for storing instructions, wherein the instructions, when executed by the processor, cause the computing device to perform operations comprising, obtaining structural variant candidates identified from whole-genome sequencing data including a pair of tumor genome data and normal tissue genome data, extracting features of each structural variant candidate based on data located within a predetermined range from each structural variant candidate in the whole-genome sequencing data, labeling each structural variant candidate with classification information based on a list of known structural variants, and training a machine learning model by using a dataset, in which features of each structural variant candidate and the labeled classification information are annotated, wherein the machine learning model receives an identification target structural variant candidate and outputs a classification of the identification target structural variant candidate.

### Brief Description of The Drawings

These and/or other aspects will become apparent and more readily appreciated from the following description of the embodiments, taken in conjunction with the accompanying drawings in which:
FIG. 1 is a diagram schematically describing a conventional structural variant identification method;
FIG. 2 is a block diagram of a genomic structural variant identification device according to an exemplary embodiment;
FIG. 3 is a diagram for describing a data structure according to an embodiment;
FIGS. 4a and 4b are diagrams exemplarily describing a method of extracting structural variant information from a structural variant candidate region according to an exemplary embodiment;
FIG. 5 is a flowchart of a training method for identifying structural variants in genomes according to an exemplary embodiment;
FIG. 6 is a diagram schematically describing structural variant identification using a trained machine learning model according to an embodiment;
FIG. 7 is a flowchart of a structural variant identification method using a trained machine learning model according to an embodiment; and
FIG. 8 is a hardware configuration diagram of a computing device according to an embodiment.

### Detailed Description

Hereinafter, with reference to the accompanying drawings, embodiments of the present invention will be described in detail so that those of ordinary skill in the art, to which the present invention pertains, can easily implement them. However, the present invention may be embodied in several different forms and is not limited to the embodiments described herein. And, in order to clearly describe the present invention in the drawings, parts irrelevant to the description are omitted, and similar reference numerals are attached to similar parts throughout the specification.

Throughout the specification, when a part "includes" a certain component, it means that other components may be further included, rather than excluding other components, unless otherwise stated. In addition, terms such as "...unit," "...or," and "module" described in the specification mean a unit that processes at least one function or operation, which may be implemented as hardware or software or a combination of hardware and software.

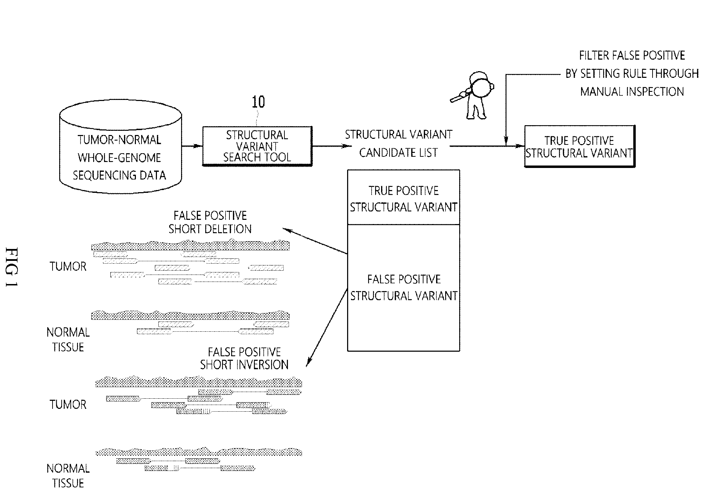

FIG. 1 is a diagram schematically illustrating a conventional structural variant identification method.

Referring to FIG. 1, as a tool 10 for searching for structural variants (SVs) in a pair of tumor and normal whole-genome sequences, DELLY, BRASS, SvABA, dRanger, and the like exist. In the structural variant search tool, when the caller calls a structural variant from the whole-genome sequence, the output structural variant candidates include significant false positives (FP) as well as true positives (TP).

Therefore, it is necessary to remove false positives from the called structural variant candidates to generate an accurate structural variant list. Until now, experts filter false positives by setting optimal filtering conditions through heuristic trial and error through manual inspection, so it took a considerable amount of time. In addition, since the filtering conditions are different depending on the sample quality and cancer type, even if it takes a considerable amount of time, accurate results cannot be provided when the filtering condition is applied to other cohorts, and it takes additional time to adjust the filtering condition for the cohort. In addition, it is difficult to find an optimal cutoff because various conditions are related, and it is difficult to explain the false positive classification criteria because it is a filtering condition based on manual inspection.

On the other hand, as machine learning-based artificial intelligence technology has been developed, analysis by applying machine learning to genomics is being attempted. For example, Google developed DeepVariant using deep neural networks to call germline-related variants. Machine learning-based methods for the discovery of somatic variants such as point mutations have been actively studied over the past few years. However, structural variants, also called genomic rearrangements, are still under-researched. This is due to the complexity of structural variants, ranging from simple deletion, duplication, inversion, insertion and translocation, to large-scale genomic rearrangements.

For example, a point mutation or a small insertion and deletion (indel) is a variant that exists in a read, which is a basic unit of sequencing data, since the exact position can be specified, a read with variants and a reference read can be examined at that position. On the other hand, since the size of the structural variant is longer than one read, the variant-supporting reads do not exist only at the breakpoint, and thus the exact position is often not specified. Therefore, a position searched by a known structural variant search tool is often inaccurate.

In the following, a method and apparatus for automatically identifying genomic structural variants with complexity using a machine learning model will be described in detail.

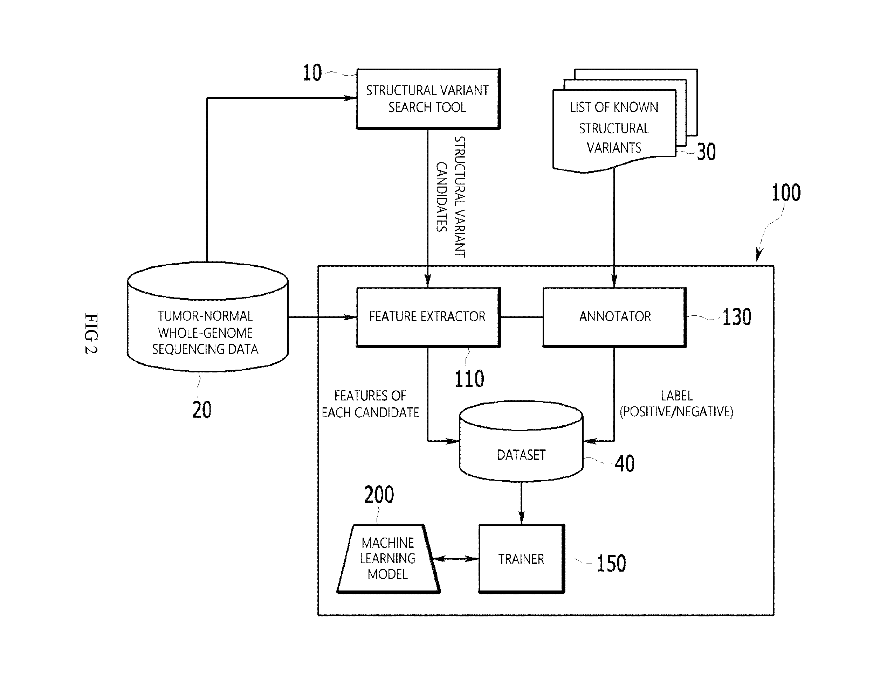

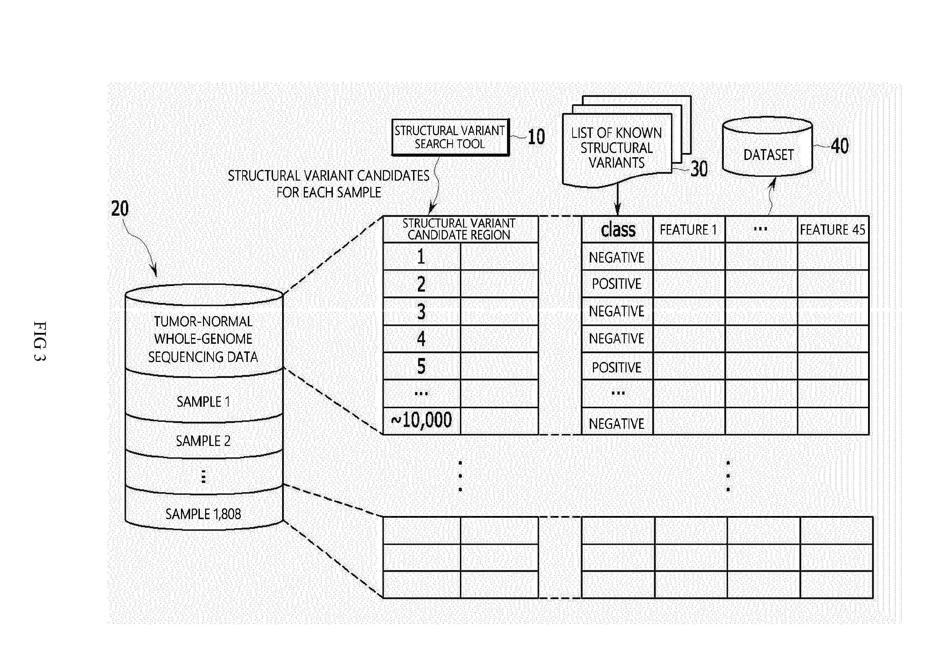

FIG. 2 is a block diagram of a genomic structural variant identification device according to an embodiment, and FIG. 3 is a diagram for describing a data structure according to an embodiment.

Referring to FIG. 2, a genomic structural variant identification device 100 (referred to simply as "device") is a computing device operated by at least one processor, and includes a feature extractor 110 for extracting the features of structural variant candidates output by a structural variant search tool 10, an annotator 130 for labeling positive (True)/negative (False) for each structural variant candidate, a trainer 150 for training a machine learning model 200 using a dataset labeled with the features. The machine learning model 200 may be implemented as various models such as a logistic regression model, a random forest model, a stochastic gradient boosting model, and the like.

The device 100 includes at least one processor, and the processor executes instructions included in a computer program, thereby performing the operations of the present disclosure. The computer program includes instructions that are described for a processor to execute the operations of the present disclosure, and may be stored in a non-transitory computer readable storage medium.

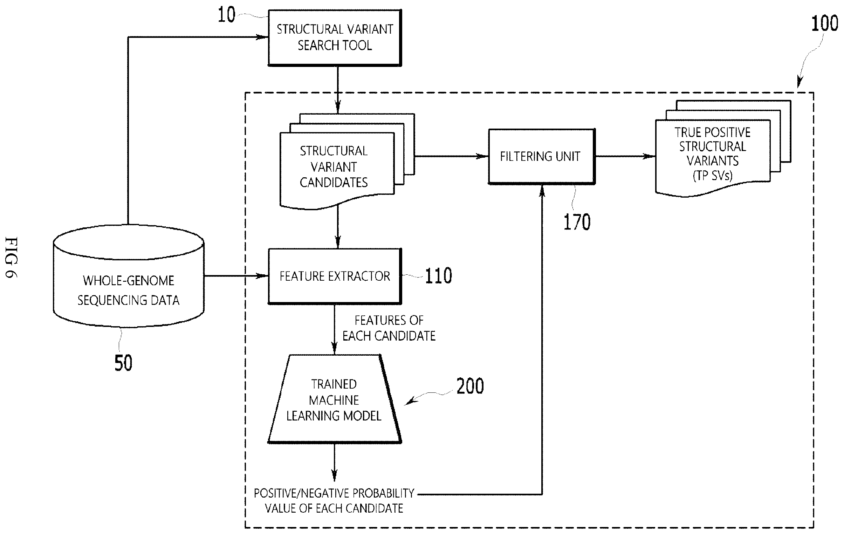

Here, the feature extractor 110, the annotator 130, the trainer 150, and the machine learning model 200 may be implemented as a computer program stored in a computer readable storage medium, and the device 100 may execute instructions included in the computer program, thereby operating the feature extractor 110, the annotator 130, the trainer 150, and the machine learning model 200. The trained machine learning model 200, the feature extractor 110, the annotator 130, the trainer 150, and the filtering unit 170 described in FIG. 6 are manufactured as a computer program and downloaded through a network, or may be sold in the form of a product, and may be installed in computing devices of various sites such as research institutes and hospitals.

It is assumed that the structural variant search tool 10 is a tool for outputting structural variant candidates by calling the putative structural variants in whole-genome sequencing, and outputs the called structural variant candidates to the device 100. In the present disclosure, it is assumed that the structural variant search tool 10 uses a known tool.

The tumor-normal whole-genome sequencing data 20 includes a pair of tumor genome data and normal tissue genome data extracted from the same person, and can be produced by short-read sequencing platforms such as Illumina. The tumor-normal whole-genome sequencing data 20 may include various types of cancer samples.

Referring to FIG. 3, the tumor-normal whole-genome sequencing data 20 constructed in the present disclosure includes a total of 1,808 samples, and may include training samples and validation samples. The training samples used in this disclosure included a total of 1,212 cancer genomes corresponding to 35 tumor types from 20 tumor tissues obtained from the international cancer genome consortium, and the validation samples include 499 cancer genomes from the same cohort and 97 cancer genomes from independent studies, and the composition of the samples can be variously adjusted.

The feature extractor 110 receives structural variant candidates searched for in the whole-genome sequencing (WGS) data by the structural variant search tool 10, and examines a surrounding region of each structural variant candidate in a pair of tumor genome data and normal tissue genome data to extract the features of each structural variant candidate. Also, the feature extractor 110 may include some features extracted from clinical data. The feature extractor 110 stores the features of each structural variant candidate as a dataset 40 for machine learning.

On the other hand, unlike point mutations or short insertions and deletions, in which the variant position is specified in one read, it is often to not know the exact position of structural variants. Therefore, the feature extractor 110 may modify the positions of the structural variant candidates called by the structural variant search tool 10 to the correct coordinates using a supplementary alignment tag (SA tag) since they may be incorrect, and merge candidates within 500 base pairs with each other in the same strand direction (e.g., 5' to 3').

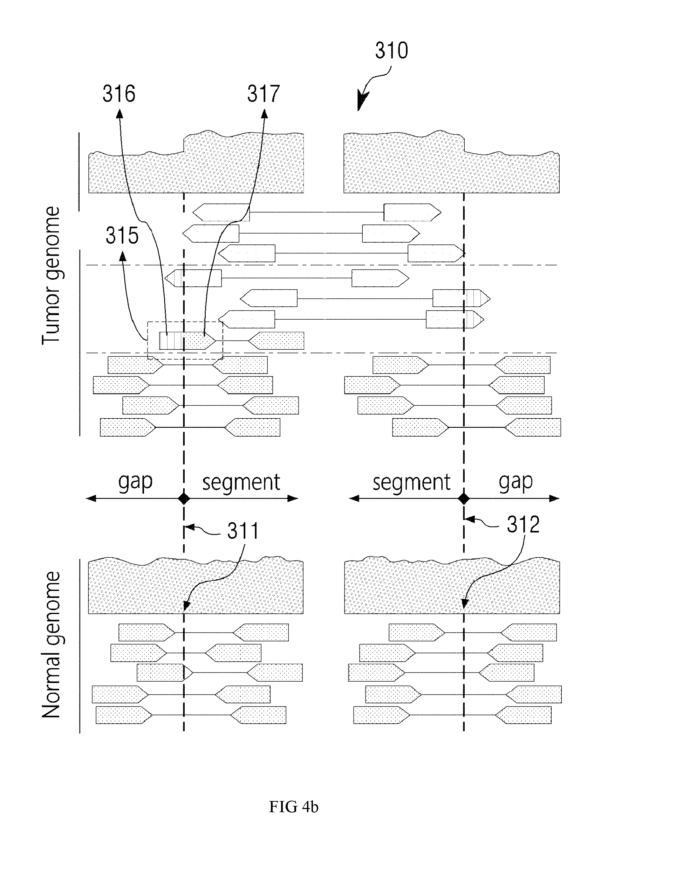

Here, the supplementary alignment tag may be one of data output as a result of the alignment tool mapping each read to a reference sequence. The supplementary alignment tag of a specific read may be information indicating another position on the reference sequence, to which the corresponding read can be mapped, in addition to the main position, to which the corresponding read is mapped on the reference sequence. For example, such as read 315 in FIG. 4b, if a particular read is a split read located at breakpoints (e.g., points 311 and 312 in FIG. 4b), the corresponding read may be mapped to two different positions or regions that are not continuous with each other on the reference sequence. Specifically, it can be analyzed that most sequences of the read located at the breakpoint (e.g., reference number 317 in FIG. 4b) is mapped to the first region of the reference sequence, and the remainder sequences of the read (e.g., reference number 316 in FIG. 4b) is mapped to the second region that is not continuous with the first region. In this case, the alignment tool may determine that the read maps to the first region on a reference sequence, but record information about the second region, to which the remaining portion of the read (e.g., reference numeral 316 in FIG. 4b) maps, as the SA tag and output it.

The feature extractor 110 may additionally use the supplementary alignment tag to more accurately determine whether any one read is a split read located at a breakpoint of the structural variant candidate, and through this, determine the position of the breakpoint of the structural variant candidate in a single base-pair resolution, thereby optimizing the region range, from which to extract features.

Referring back to FIG. 3, when a plurality of structural variant candidates are searched for by each sample in the structural variant search tool 10, each structural variant candidate may be displayed as a junction between two breakpoints (breakpoint1 and breakpoint2) in the genome data. Here, the two breakpoints are aligned according to chromosome and position.

The feature extractor 110 extracts the features of the tumor genome data different from the normal tissue genome data by examining the breakpoints (breakpoint1 and breakpoint2) of each structural variant candidate in the tumor genome data and the normal tissue genome data.

Structural variants have features, in which a sufficient number of variant-supporting reads and many split reads by breakpoints are included. On the other hand, short deletions or short inversions, which are also found in normal tissue genomes, cause errors and are searched as a false-positive structural variant. In addition, false-positive structural variants are found in the noise region characterized by high read depth, low mapping quality, and the like. In order to find the features of this structural variant, the feature extractor 110 extracts the number of variant-supporting reads (Features 13 and 14 in Table 1), the number of split reads (Features 16 and 17 in Table 1), the number of split reads with supplementary alignment tag (Feature 18 in Table 1), mapping quality (Features 22 and 23 in Table 1), read depth change (Features 24 and 25 in Table 1), the number of background noise reads (Features 33, 34, 37, 38 in Table 1), and the number of samples, in which the same variant is detected among the panel of normal tissue genome data (Feature 45 in Table 1), and the like.

Specifically, the feature extractor 110 may store 45 features defined as shown in Table 1, and extract a value indicated by each feature using user-defined functions set to find the features. Among the 45 features, tumor histology, whole-genome duplication status, tumor purity, and tumor ploidy of the tumor genome may be extracted from clinical data of the sample. For the remaining 41 features, the feature extractor 110 examines the surrounding regions of each structural variant candidate in the whole-genome sequencing data, and extracts a value corresponding to each feature (binary value, number of counts, p-value, frequency, quality, ratio, distance, etc.).

**[Table 1]**

| No. | Feature | Description |
|---|---|---|
| 1 | tumor histology | tumor tissue (20 categories): Biliary, Bladder, Bone_SoftTissue, Breast, Cervix, CNS, Colon_Rectum, Esophagus, Head_Neck, Hematologic, Kideny, Liver, Lung, Ovary, Pancreas, Prostate, Skin, Stomach, Thyroid, Uterus |
| 2 | whole-genome duplication status | feature indicating whether tumor whole-genome was duplciated (binary value): TRUE, FALSE |
| 3 | structural variant type | structural variant type (4 categories): deletion, duplication, inversion, and translocation |
| 4 | positional variation at breakpoint1 | whether or not the mapping position of the variant-supporting reads at the breakpoint1 of the tumor genome data is variable (binary value): TRUE, FALSE |
| 5 | positional variation at breakpoint2 | whether or not the mapping position of the variant-supporting reads at the breakpoint2 of the tumor genome data is variable (binary value): TRUE, FALSE |
| 6 | microhomology | whether presence or absence of microhomologous DNA sequences at two breakpoints (binary value): TRUE, FALSE |
| 7 | tumor purity | tumor cell fraction of tumor genome data |
| 8 | tumor ploidy | tumor ploidy of tumor genome data |
| 9 | reference read count at breakpoint1 in tumor WGS | number of reference reads at breakpoint1 in tumor genome data |
| 10 | reference read count at breakpoint2 in tumor WGS | number of reference reads at breakpoint2 in tumor genome data |
| 11 | reference read count at breakpoint1 in normal WGS | number of reference reads at breakpoint1 in normal tissue genome data |
| 12 | reference read count at breakpoint2 in normal WGS | number of reference reads at breakpoint2 in normal tissue genome data |
| 13 | total variant-supporting read count in tumor WGS | number of variant-supporting reads at two breakpoints in tumor genome data |
| 14 | total variant-supporting read count in normal WGS | number of variant-supporting reads at two breakpoints in normal tissue genome data |
| 15 | significance of variant fraction | p-value of Fisher's exact test for a 2x2 table of reference read and number of variant-supporting reads in tumor and normal tissue genome data |
| 16 | split read count in tumor WGS | number of split reads at two breakpoints in tumor genome data |
| 17 | split read count in normal WGS | number of split reads at two breakpoints in normal tissue genome data |
| 18 | count of split reads with supplementary alignment tag in tumor WGS | number of split reads with supplementary alignment tag at two breakpoints in tumor genome data |
| 19 | same clipped read count in normal WGS | number of reads with the same clipped sequence as split read at two breakpoints in normal tissue genome data |
| 20 | variant allele fraction at breakpoint1 in tumor WGS | fraction of variant-supporting reads at breakpoint1 in tumor genome data |
| 21 | variant allele fraction at breakpoint2 in tumor WGS | fraction of variant-supporting reads at breakpoint2 in tumor genome data |
| 22 | median mapping quality at breakpoint1 in tumor WGS | median value of mapping quality of variant-supporting reads at breakpoint1 in tumor genome data |
| 23 | median mapping quality at breakpoint2 in tumor WGS | median value of mapping quality of variant-supporting reads at breakpoint2 in tumor genome data |
| 24 | depth ratio change at breakpoint1 in tumor WGS | read depth change at breakpoint1 in tumor genome data |
| 25 | depth ratio change at breakpoint2 in tumor WGS | read depth change at breakpoint2 in tumor genome data |
| 26 | new mate count at breakpoint1 in tumor WGS | number of new structural variants extracted from supplementary alignment tag of variant-supporting read at breakpoint1 of tumor genome data |
| 27 | new mate count at breakpoint2 in tumor WGS | number of new structural variants extracted from supplementary alignment tag of variant-supporting read at breakpoint2 of tumor genome data |
| 28 | neo mate count at breakpoint2 in tumor WGS | number of structural variants inferred from reads corresponding to Feature 26 |
| 29 | neo mate count at breakpoint2 in tumor WGS | number of structural variants inferred from reads corresponding to Feature 27 |
| 30 | distance between breakpoint1 and 2 | distance between breakpoint 1 and breakpoint 2, which is set to the size of chromosome 1 if structural variant is translocation |
| 31 | total clipped read count at breakpoint1 in tumor WGS | number of all clipped reads at breakpoint1 in tumor genome data |
| 32 | total clipped read count at breakpoint2 in tumor WGS | number of all clipped reads at breakpoint2 in tumor genome data |
| 33 | total clipped read count at breakpoint1 in normal WGS | number of all clipped reads at breakpoint1 in normal tissue genome data |
| 34 | total clipped read count at breakpoint2 in normal WGS | number of all clipped reads at breakpoint2 in normal tissue genome data |
| 35 | other discordant read pair cluster at breakpoint1 in tumor WGS | number of discordant read pair clusters other than variant-supporting reads at breakpoint1 in tumor genome data |
| 36 | other discordant read pair cluster at breakpoint2 in tumor WGS | number of discordant read pair clusters other than variant-supporting reads at breakpoint2 in tumor genome data |
| 37 | other discordant read pair cluster at breakpoint1 in normal WGS | number of discordant read pair clusters other than variant-supporting reads at breakpoint1 in normal tissue genome data |
| 38 | other discordant read pair cluster at breakpoint2 in normal WGS | number of discordant read pair clusters other than variant-supporting reads at breakpoint2 in normal tissue genome data |
| 39 | read depth at breakpoint1 in normal WGS | read depth at breakpoint1 in normal tissue genome data |
| 40 | read depth at breakpoint2 in normal WGS | read depth at breakpoint2 in normal tissue genome data |
| 41 | GC content at breakpoint1 | fraction of G and C bases at ±50 bp of breakpoint1 |
| 42 | GC content at breakpoint2 | fraction of G and C bases at ±50 bp of breakpoint2 |
| 43 | soft-masked base at breakpoint1 | fraction of soft-masked bases at ±50bp of breakpoint 1 |
| 44 | soft-masked base at breakpoint2 | fraction of soft-masked bases at ±50bp of breakpoint 2 |
| 45 | panel of normal samples | number of samples, in which the same variant is detected among normal tissue genome data belonging to training data |

In Table 1, tumor histology, whole-genome duplication status, structural variant type, positional variant at breakpoint1, positional variant at breakpoint2, and whether presence or absence of microhomologous DNA sequences at two breakpoints (microhomology) are categorical variables, and the number converted using one-hot encoding may be described. The remaining 39 features are continuous variables, and the number corresponding to each feature may be described.

The number of samples, in which the same variant is detected among the panel of normal tissue genome data, which is Feature 45 of Table 1, is extracted from the panel of normal tissue genome data included in the training cohort. As the number of data increases, the number of objects to compare whether the structural variant is true or an artifact increases. Thus, the larger the data, the better the performance.

On the other hand, the extracted features may not represent the structural variant well due to inaccuracy in the structural variant detection by a structural variant search tool. To solve this problem, the feature extractor 110 uses the supplementary alignment tag to more accurately determine the split read located at the breakpoint of the structural variant candidate, and through this, to determine the position of the breakpoint of the structural variant candidates in a single base-pair resolution, thereby optimizing the range of candidate regions from which to extract features.

In addition, since there are many structural variant candidate regions, the time required for feature extraction is considerable. To this end, the feature extractor 110 may detect a noise region, in which noise is greater than or equal to a reference level in the genome data, and omit feature extraction of the detected noise region. Through this, it is possible to increase the feature extraction speed of structural variant candidates.

In particular, since the size of the structural variant is longer than one read, the variant-supporting reads do not exist only at the breakpoint, and thus it is often not to be specified the exact position. To solve this problem, the feature extractor 110 may examine the surrounding area of each structural variant candidate through user-defined functions set to extract the features of Table 1.

In this case, the search range for the surrounding region of the breakpoint may be set differently depending on the type of structural variant or the extracted features. In particular, among the features of Table 1, whether the mapping position of the variant-supporting reads in the surrounding region of the breakpoint is variable (Features 4 and 5 in Table 1), whether presence or absence of a microhomologous DNA sequences at the two breakpoints (Feature 6 in Table 1), the number of reference reads at the two breakpoints (Features 9-12 in Table 1), the number of variant-supporting reads at the two breakpoints (Features 13 and 14 in Table 1), the number of split reads at the two breakpoints (Features 16 and 17 in Table 1), and the number of split reads with supplementary alignment tags at the two breakpoints (Features 18 in Table 1), the number of reads with the same clipped sequence as the split read in the normal tissue genome data at the two breakpoints (Feature 19 in Table 1), the median value of mapping quality of the variant-supporting reads at the breakpoint in the tumor genome data (Features 22 and 23 in Table 1), changes in read depth at the breakpoint in the tumor genome data (Features 24 and 25 in Table 1), the number of new structural variants extracted from the supplementary alignment tags of the variant-supporting reads at the breakpoint in the tumor genome data, and the number of inferred structural variants (Features 26-29 in Table 1), the number of all clipped reads at the breakpoint (Features 31-34 in Table 1), the number of discordant read pair clusters at the breakpoint other than variant-supporting reads (Feature 35-38 in Table 1), the read depth at the breakpoint in the normal tissue genome data (Features 39 and 40 in Table 1), etc. are extracted, so that structural variants with complexity can be accurately identified.

In some embodiments, in particular, in the case of extracting the number of variant-supporting reads (Features 13 and 14 in Table 1), the number of split reads (Features 16 and 17 in Table 1), the number of split reads with supplementary alignment tags (Feature 18 in Table 1), and the number of reads with the same clipped sequence as the split read in normal tissue genome data (Feature 19 in Table 1), the range to examine the surrounding region of the breakpoint may be set as follows.

First, from the breakpoint (e.g., positions 311 and 312 in FIG. 4b) to the direction, in which structural variant-supporting reads are located (e.g., the direction indicated as segment in FIG. 4b), it can be set to examine up to the average insert size of the entire paired-end reads of the processing target whole-genome sequencing data or further. This is because the size of the structural variant is longer than one read, so there may be variant-supporting reads located in the segment direction of the two breakpoints without overlapping any other breakpoints.

On the other hand, in consideration of the possibility that the data regarding the position of structural variant candidate output by the structural variant search tool 10 is inaccurate, it may be desirable to examine a predetermined range also in the opposite direction of the structural variant-supporting reads (e.g., the direction marked as gap in FIG. 4b) from the breakpoint (e.g., the positions 311 and 312 in FIG. 4b). Specifically, it may be set to examine, for example, 200 base pairs in the gap direction from the breakpoint. It should be noted that if a range wider than 200 base pairs is examined in the gap direction from the breakpoint, there is a possibility that data resulting from structural variants other than the target structural variant candidate near the breakpoint or other discordant reads are examined.

Referring back to FIG. 3, the annotator 130 labels each structural variant candidate as positive (True)/negative (False) for the structural variant candidates searched for in the whole-genome sequencing data, and stores it as a dataset 40 for machine learning. In this case, the annotator 130 may refer to a list of known structural variants 30, and label it as positive if each structural variant candidate exists in the structural variant list 30, otherwise label it as negative.

The features of each structural variant candidate extracted by the feature extractor 110 and the classification classes of each structural variant candidate classified by the annotator 130 are stored in the dataset 40 as shown in FIG. 3.

The trainer 150 trains the machine learning model 200 using the dataset 40. The trainer 150 trains the machine learning model 200 to classify the corresponding structural variant candidate into a classification class from features of each structural variant candidate. The machine learning model 200 may be implemented as various models such as a logistic regression model, a random forest model, a stochastic gradient boosting model, and the like.

The trainer 150 may use the validation samples included in the tumor-normal whole-genome sequencing data 20 to verify the performance of the machine learning model 200 and then complete the training. The trainer 150 may evaluate performance using a precision-recall curve.

The trained machine learning model 200 may receive features of each structural variant candidate and output a probability value between 0 and 1. The probability value of each structural variant candidate is determined as positive or negative according to the set cutoff value. A structural variant candidate that is negative may be filtered out as false positive.

When the machine learning model 200 according to the present disclosure is used, about 5,800 structural variants not found in previous studies were newly discovered in addition to 32,134 structural variants reported in 249 test samples.

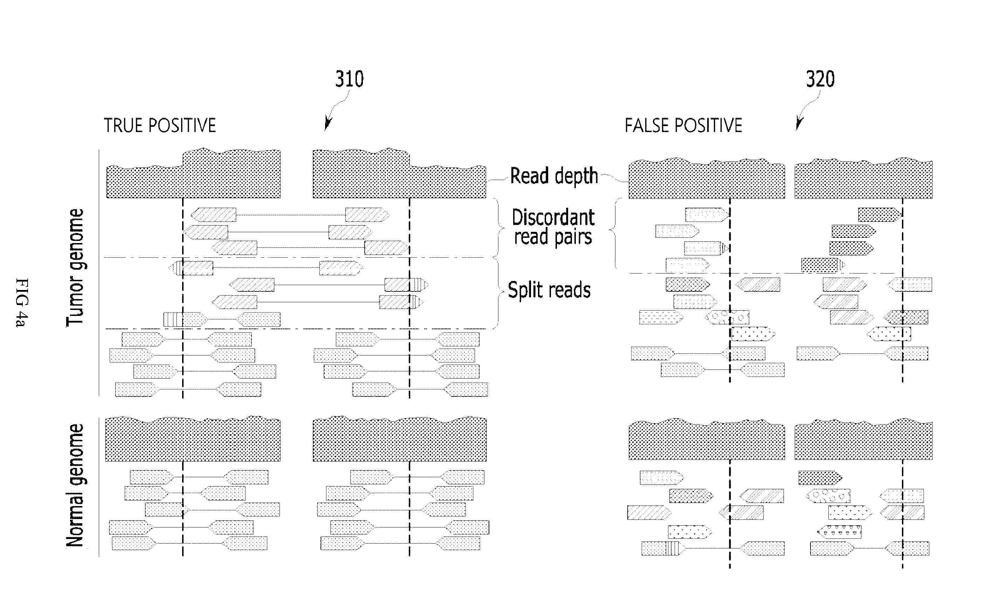

FIGS. 4a and 4b are diagrams exemplarily describing a method of extracting structural variant information in a structural variant candidate region according to an exemplary embodiment.

Referring to FIG. 4a, the feature extractor 110 extracts features defined for classifying false positives for each structural variant candidate called by the structural variant search tool 10. In FIG. 4a, reads of sequencing data are colored with their mate reads, and thus non-gray reads indicate discordant read pairs.

For example, when a true positive structural variant 310 and a false positive structural variant 320, in which a duplication event exists, are called, the feature extractor 110 extracts values of variant-supporting reads in tumor genome data, split reads, mapping quality, variant-supporting reads in normal tissue genome data, read depth change, and background noise reads, as shown in Table 2, and in addition, may extract values of a plurality of features defined in Table 1. If desired, discordant read pairs can be calculated.

**[Table 2]**

| Extracted Feature | True Positive Structural Variant | False Positive Structural Variant |
|---|---|---|
| Variant-supporting reads | 7 | 4 |
| Split reads | 4 | 0 |
| Mapping quality | 56 | 20 |
| Variant-supporting reads in normal | 0 | 1 |
| Read depth change | 1.8 | 0.9 |
| Background noise reads | 0 | 8 |

The feature extractor 110 stores data filled with features of each structural variant candidate as a dataset 40 for machine learning.

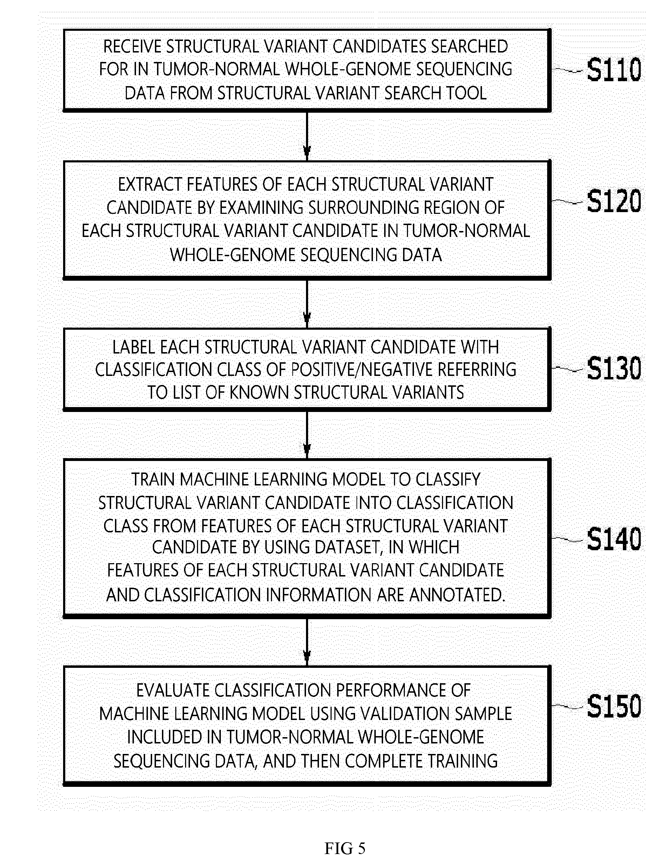

FIG. 5 is a flowchart of a training method for identifying structural variants in genomes according to an exemplary embodiment.

Referring to FIG. 5, the device 100 receives structural variant candidates searched for in tumor-normal whole-genome sequencing data 20 from the structural variant search tool 10 (S110).

The device 100 extracts the features of each structural variant candidate by examining a surrounding region of each structural variant candidate in the tumor-normal whole-genome sequencing data 20 (S120). The device 100 extracts a value corresponding to each feature by examining the vicinity of two breakpoints (breakpoint1 and breakpoint2) for each structural variant candidate. The number of variant-supporting reads, the number of split reads with supplementary alignment tag, the number of split reads, mapping quality, read depth change, the number of background noise reads, and the number of samples, in which the same variant is detected among the panel of normal tissue genome data, etc. are extracted as the features. At this time, the device 100 may additionally receive tumor histology, whole-genome duplication status, tumor purity, and tumor ploidy of the tumor genome from the clinical data of the sample.

The device 100 labels each structural variant candidate with a classification class of positive (True)/negative (False) with reference to the list of known structural variants 30 (S130). The device 100 may label it as positive if each structural variant candidate has the same direction as the structural variant in the structural variant list 30 and exits within a distance of 500 base pairs, otherwise may label it as negative.

The device 100 trains the machine learning model 200 to classify the structural variant candidate into a classification class from the features of each structural variant candidate using a dataset, in which classification classes are mapped to the features of each structural variant candidate (S140). The machine learning model 200 may receive features of each structural variant candidate and output a classification probability of positive or negative (a probability value between 0 and 1).

The trainer 150 may verify the classification performance of the machine learning model 200 by using the validation samples included in the tumor-normal whole-genome sequencing data 20, and then complete the training (S150). In this case, the trainer 150 may determine a cutoff value for classifying into the labeled positive or negative according to a probability value between 0 and 1 output from the machine learning model 200 based on the classification performance evaluation.

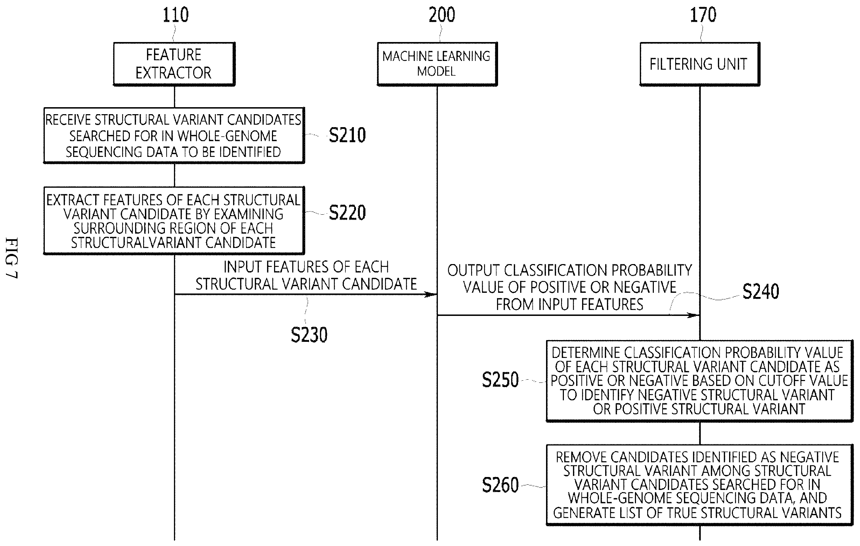

FIG. 6 is a diagram schematically describing structural variant identification using a trained machine learning model according to an embodiment, and FIG. 7 is a flowchart of a structural variant identification method using the trained machine learning model according to an embodiment.

Referring to FIGS. 6 and 7, in order to identify structural variants in the whole-genome sequencing data to be identified, the device 100 may use the feature extractor 110 and the trained machine learning model 200, and additionally use a filtering unit 170. In this case, the feature extractor 110, the filtering unit 170, and the trained machine learning model 200 may be implemented as a computer program, and may be driven in any computing device, in which they are installed. In addition, the annotator 130 and the trainer 150 of FIG. 2 may also be implemented as computer programs, and may be driven in a computing device, in which they are installed. In this case, the users can re-train the trained machine learning model 200 with the genome data of their cohort.

The feature extractor 110 receives structural variant candidates searched for in the whole-genome sequencing data 50 from the structural variant search tool 10. The feature extractor 110 extracts the features of each structural variant candidate used for training the machine learning model 200 by examining the surrounding region of each structural variant candidate. The feature extractor 110 inputs the features of each structural variant candidate into the trained machine learning model 200.

The trained machine learning model 200 outputs a classification probability value of positive or negative from the input features.

Then, the filtering unit 170 may classify the classification probability value of each structural variant candidate output from the trained machine learning model 200 as negative or positive based on the cutoff value to identify the true structural variant. In addition, the filtering unit 170 may filter and remove structural variant candidates classified as negative among the structural variant candidates, and generate a list of true structural variants including True Positive Structural Variants (TP SVs) classified as positive.

Referring to FIG. 7, the feature extractor 110 receives structural variant candidates searched for in the whole-genome sequencing data 50 from the structural variant search tool 10 (S210).

The feature extractor 110 extracts the features of each structural variant candidate by examining the surrounding region of each structural variant candidate (S220). The feature extractor 110 may examine the surrounding region of each structural variant candidate through user-defined functions set to extract the features of Table 1. In this case, the range to be examined in the vicinity of the breakpoint may be set differently according to the type of a structural variant or the extracted feature.

The feature extractor 110 inputs the features of each structural variant candidate into the trained machine learning model 200 (S230).

The trained machine learning model 200 outputs a classification probability value of positive or negative from the input features (S240).

The filtering unit 170 determines the classification probability value of each structural variant candidate output from the trained machine learning model 200 as negative or positive based on the cutoff value, and identifies it as a negative structural variant or a positive structural variant (S250).

The filtering unit 170 removes candidates identified as negative structural variants from among the structural variant candidates searched for in the whole-genome sequencing data 50, and generates a list of true structural variants (S260). Since the structural variant candidates searched by the structural variant search tool 10 contain false positives, the filtering unit 170 determines that structural variant candidates classified as negative by the machine learning model 200 among the structural variant candidates as false positives and removes them.

As such, according to the embodiment, various structural variants in the genome can be accurately detected through the machine learning model, and an accurate structural variant list can be generated by removing false positives, thereby contributing to precision oncology.

According to an embodiment, when a machine learning model trained with the standardized features of the structural variant candidates is used, false positives can be quickly removed from the structural variant candidates.

According to the embodiment, when a machine learning model trained with standardized features is used, the time required for experts setting the optimal filtering conditions through heuristic trial and error through manual inspection, and filtering false positives can be significantly reduced.

According to the embodiment, scalability can be increased through standardized structural variant search using a machine learning model, and manual curation by experts is unnecessary, thereby reducing cost and time.

According to an embodiment, it can be applied to various cohorts through simple re-training of the machine learning model, and an optimal cutoff can be found based on classification performance.

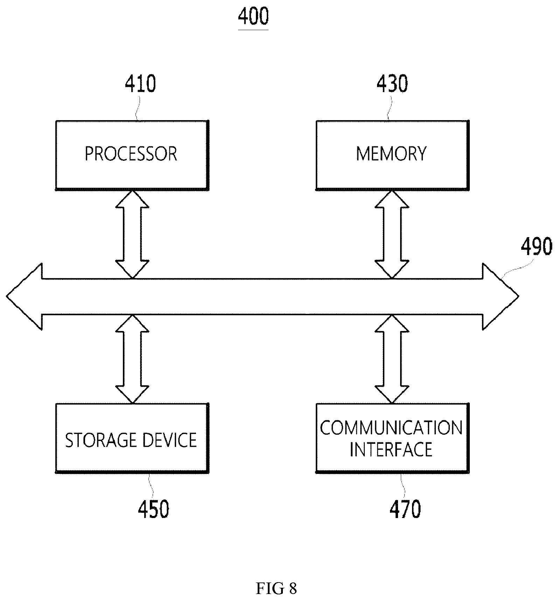

FIG. 8 is a hardware configuration diagram of a computing device according to an embodiment.

Referring to FIG. 8, the device 100 may be implemented as a computing device 400 operated by at least one processor. Alternatively, each of the feature extractor 110, the annotator 130, the trainer 150, the filtering unit 170, and the machine learning model 200 may be mounted on the computing device 400 operated by at least one processor.

The computing device 400 includes one or more processors 410, a memory 430 for loading a computer program executed by the processor 410, a storage device 450 for storing computer programs and various data, a communication interface 470, and a bus 490 connecting them. In addition, the computing device 400 may further include various components.

The processor 410 is a device for controlling the operation of the computing device 400, and may be various types of processors that process instructions included in a computer program, and may include, for example, a central processing unit (CPU), a microprocessor unit (MPU), a micro controller unit (MCU), a graphic processing unit (GPU), or any type of processor well known in the art of the present disclosure.

The memory 430 stores various data, instructions and/or information. The memory 430 may load a corresponding computer program from the storage device 450 so that the instructions described to execute the operations of the present disclosure are processed by the processor 410. The memory 430 may be, for example, read only memory (ROM), random access memory (RAM), or the like.

The storage device 450 may non-temporarily store computer programs and various data. The storage device 450 may include a non-volatile memory, such as a read only memory (ROM), an erasable programmable ROM (EPROM), an electrically erasable programmable ROM (EEPROM), a flash memory, a hard disk, a removable disk, or any well-known computer-readable recording medium in the art, to which the present disclosure pertains.

The communication interface 470 may be a wired/wireless communication module supporting wired/wireless communication.

The bus 490 provides a communication function between the components of the computing device 400.

The computer program includes instructions executed by the processor 410, and is stored in a non-transitory computer readable storage medium, wherein the instructions have the processor 410 execute the operation of the present disclosure. The computer program may be downloaded over a network or sold as a product.

The computer program for training may comprise instructions described to execute the step of receiving, from the structural variant search tool, structural variant candidates searched for in whole-genome sequencing data including a pair of tumor genome data and normal tissue genome data, the step of extracting features of each structural variant candidate by examining the surrounding regions of each structural variant candidate in the whole-genome sequencing data, the step of labeling each structural variant candidate with a classification class of positive or negative with reference to a list of known structural variant candidates, and the step of training the machine learning model to classify the corresponding structural variant candidate into a classification class from the features of each structural variant candidate using the dataset, in which the classification classes are mapped to the features of each structural variant candidate. The computer program for training may further comprise instructions described to execute the step of using the validation samples included in the whole-genome sequencing data to verify the classification performance of the machine learning model, and then completing the training, and the step of determining a cutoff value for classifying the probability value output from the machine learning model as positive or negative based on the evaluation of the classification performance of the machine learning model.

In this case, the computer program for training may store features extracted from each structural variant candidate. The features may include the number of variant-supporting reads, the number of split reads with supplementary alignment tag, the number of split reads, mapping quality, read depth change, the number of background noise reads, and the number of samples, in which the same variant is detected among the panel of normal tissue genome data. The features may further include tumor histology, whole-genome duplication status, tumor purity, and tumor ploidy of the tumor genome extracted from clinical data.

Meanwhile, the computer program for training may include a machine learning model that receives features of each structural variant candidate and outputs a classification probability value of positive or negative.

After being trained, the computer program distributed for structural variant identification may include a first computer program trained to classify a corresponding structural variant candidate into a classification class from features of each structural variant candidate, and a second computer program. The second computer program may include instructions described to receive structural variant candidates searched for in the whole-genome sequencing data to be identified, examine the surrounding area of each structural variant candidate in the whole-genome sequencing data to extract the features of each structural variant candidate, then input the features of each structural variant candidate into the machine learning model to obtain a classification probability value of positive or negative for the structural variant candidate, and determine the classification probability value as negative or positive based on the set cutoff value to identify each structural variant candidate as a negative or positive structural variant.

The first computer program may be a machine learning model implemented as at least one of a logistic regression model, a random forest model, and a stochastic gradient boosting model.

The second computer program may further include instructions for removing the structural variant candidates identified as negative structural variant candidates from among the structural variant candidates searched by the structural variant search tool to generate a list of true structural variants. The second computer program stores features extracted from each structural variant candidate, wherein the features may include the number of variant-supporting reads, the number of split reads with supplementary alignment tag, the number of split reads, mapping quality, read depth change, the number of background noise reads, and the number of samples, in which the same variant is detected among the panel of normal tissue genome data. The second computer program may further include instructions for additionally extracting features of each structural variant candidate from clinical data, and the additionally extracted features may include tumor histology, whole-genome duplication status, tumor purity in the sample, and tumor ploidy of the tumor genome.

The embodiment of the present invention described above is not implemented only through the apparatus and method, but may be implemented through a program for realizing a function corresponding to the configuration of the embodiment of the present invention or a recording medium, in which the program is recorded.

Although the embodiments of the present invention have been described in detail above, the scope of the present invention is not limited thereto, and various modifications and improved forms of the present invention provided by those skilled in the art using the basic concept of the present invention as defined in the following claims are also within the scope of the right.

## Claims

1. A method performed by a computing device for identifying structural variants in genomes comprising:
obtaining structural variant candidates identified from whole-genome sequencing data including a pair of tumor genome data and normal tissue genome data;
extracting features of each structural variant candidate based on data located within a predetermined range from each structural variant candidate in the whole-genome sequencing data; and
labeling each structural variant candidate with classification information based on a list of known structural variants, wherein each structural variant candidate is labeled as positive if said structural variant candidate exists in the list of known structural variants or otherwise said structural variant candidate is labeled as negative; and
training a machine learning model by using a dataset, in which the extracted features of each structural variant candidate and the labeled classification information are stored,
wherein the machine learning model receives an identification target structural variant candidate and outputs a classification of the identification target structural variant candidate, wherein the machine learning model receives features of the identification target structural variant candidate and outputs a probability value for classifying the identification target structural variant candidate as positive or negative;
**characterized in that**
extracting features of each structural variant candidate comprises:
obtaining data of a first length or more in a direction, in which variant-supporting reads of said structural variant candidate are located, from a breakpoint associated with said structural variant candidate among the structural variant candidates in the whole-genome sequencing data, and
wherein the first length is an average insert size of the whole-genome sequencing data.

2. The method of claim 1 further comprises,
modifying an estimated position of a first structural variant candidate based on supplementary alignment (SA) tag information associated with the first structural variant candidate among the structural variant candidates before extracting features of each structural variant candidate.

3. The method of claim 1 further comprises,
generating a list of true structural variants by removing structural variant candidates identified as the negative structural variant from among the obtained structural variant candidates.

4. The method of claim 1, wherein modifying the estimated position of the first structural variant candidate comprises identifying a first region and a second region on a reference sequence, to which the first structural variant candidate is mapped,
wherein the first region and the second region are regions that are not adjacent to each other.

5. The method of claim 1, wherein modifying the estimated position of the first structural variant candidate comprises determining a position of a breakpoint associated with the first structural variant candidate.

6. The method of claim 1, wherein extracting features of each structural variant candidate based on data located within a predetermined range from each structural variant candidate comprises,
obtaining data of a second length or less in a direction opposite to variant-supporting reads of a first structural variant candidate from a breakpoint associated with the first structural variant candidate among the structural variant candidates in the whole-genome sequencing data.

7. The method of claim 6, wherein the second length is 200 base pairs.

8. The method of claim 1, wherein extracting the features of each structural variant candidate comprises,
extracting, for each structural variant candidate, a number of variant-supporting reads, a number of split reads with supplementary alignment tag, a number of split reads, mapping quality, read depth change, a number of background noise reads, and a number of samples, in which the same variant is detected among normal tissue genome data.

9. The method of claim 1, wherein extracting the features of each structural variant candidate comprises,
extracting, for each structural variant candidate, tumor histology, whole-genome duplication status, tumor purity in a sample, and tumor ploidy of a tumor genome from clinical data.

10. The method of claim 1, wherein the machine learning model receives features of the identification target structural variant candidate and outputs a probability value for classifying the identification target structural variant candidate as positive or negative.

11. The method of claim 1 further comprises,
evaluating classification performance of the machine learning model by using the validation samples included in the whole-genome sequencing data; and
determining a cutoff value for determining whether a probability value output from the machine learning model indicates positive or negative based on the classification performance evaluation result of the machine learning model.

12. The method of claim 1, wherein obtaining structural variant candidates identified from the whole-genome sequencing data comprises,
inputting the whole-genome sequencing data into a structural variant search tool; and
obtaining structural variant candidates output by the structural variant search tool.

## Patentansprüche

1. Verfahren, das durch eine Rechenvorrichtung zum Identifizieren von Strukturvarianten in Genomen durchgeführt wird, Folgendes umfassend:
Erhalten von Strukturvariantenkandidaten, die aus Ganzgenom-Sequenzierungsdaten identifiziert wurden, einschließlich eines Paars von Tumor-Genomdaten und Normalgewebe-Genomdaten;
Extrahieren von Merkmalen jedes Strukturvariantenkandidaten basierend auf Daten, die sich innerhalb eines vorbestimmten Bereichs von jedem Strukturvariantenkandidaten in den Ganzgenom-Sequenzierungsdaten befinden; und
Kennzeichnen jedes Strukturvariantenkandidaten mit Klassifizierungsinformationen basierend auf einer Liste bekannter Strukturvarianten, wobei jeder Strukturvariantenkandidat als positiv markiert wird, wenn der Strukturvariantenkandidat in der Liste bekannter Strukturvarianten existiert, oder anderweitig der Strukturvariantenkandidat als negativ markiert wird; und
Trainieren eines Machine-Learning-Modells unter Verwendung eines Datensatzes, in dem die extrahierten Merkmale jedes Strukturvariantenkandidaten und die markierten Klassifizierungsinformationen gespeichert werden, wobei das Machine-Learning-Modell einen Identifikationsziel-Strukturvariantenkandidaten empfängt und eine Klassifizierung des Identifikationsziel-Strukturvariantenkandidaten ausgibt, wobei das Machine-Learning-Modell Merkmale des Identifikationsziel-Strukturvariantenkandidaten empfängt und einen Wahrscheinlichkeitswert zum Klassifizieren des Identifikationsziel-Strukturvariantenkandidaten als positiv oder negativ ausgibt;
**dadurch gekennzeichnet, dass**
das Extrahieren von Merkmalen jedes Strukturvariantenkandidaten Folgendes umfasst:
Erhalten von Daten einer ersten Länge oder mehr in einer Richtung, in der variantenunterstützende Lesevorgänge des Strukturvariantenkandidaten lokalisiert sind, von einem Bruchpunkt, der mit dem Strukturvariantenkandidaten unter den Strukturvariantenkandidaten in den Ganzgenom-Sequenzierungsdaten assoziiert ist, und
wobei die erste Länge eine durchschnittliche Insert-Größe der Ganzgenom-Sequenzierungsdaten ist.

2. Verfahren nach Anspruch 1, das ferner Folgendes umfasst:
Modifizieren einer geschätzten Position eines ersten Strukturvariantenkandidaten basierend auf Supplementary Alignment (SA)-Tag-Informationen, die mit dem ersten Strukturvariantenkandidaten assoziiert sind, unter den Strukturvariantenkandidaten vor dem Extrahieren von Merkmalen jedes Strukturvariantenkandidaten.

3. Verfahren nach Anspruch 1, das ferner Folgendes umfasst:
Erzeugen einer Liste echter Strukturvarianten durch Entfernen von Strukturvariantenkandidaten, die als die negative Strukturvariante identifiziert wurden, aus den erhaltenen Strukturvariantenkandidaten.

4. Verfahren nach Anspruch 1, wobei das Modifizieren der geschätzten Position des ersten Strukturvariantenkandidaten das Identifizieren eines ersten Bereichs und eines zweiten Bereichs auf einer Referenzsequenz umfasst, auf die der erste Strukturvariantenkandidat abgebildet ist, wobei der erste Bereich und der zweite Bereich Bereiche sind, die nicht aneinander angrenzen.

5. Verfahren nach Anspruch 1, wobei das Modifizieren der geschätzten Position des ersten Strukturvariantenkandidaten das Bestimmen einer Position eines Haltepunkts umfasst, der mit dem ersten Strukturvariantenkandidaten assoziiert ist.

6. Verfahren nach Anspruch 1, wobei das Extrahieren von Merkmalen jedes Strukturvariantenkandidaten basierend auf Daten, die sich innerhalb eines vorbestimmten Bereichs von jedem Strukturvariantenkandidaten befinden, Folgendes umfasst:
Erhalten von Daten einer zweiten Länge oder weniger in einer Richtung entgegengesetzt zu variantenunterstützenden Lesevorgängen eines ersten Strukturvariantenkandidaten von einem Bruchpunkt, der mit dem ersten Strukturvariantenkandidaten assoziiert ist, unter den Strukturvariantenkandidaten in den Ganzgenom-Sequenzierungsdaten.

7. Verfahren nach Anspruch 6, wobei die zweite Länge 200 Basispaare beträgt.

8. Verfahren nach Anspruch 1, wobei das Extrahieren der Merkmale jedes Strukturvariantenkandidaten Folgendes umfasst:
Extrahieren, für jeden Strukturvariantenkandidaten, einer Anzahl von variantenunterstützenden Lesevorgängen, einer Anzahl von geteilten Lesevorgängen mit Supplementary Alignment-Tag, einer Anzahl von geteilten Lesevorgängen, einer Abbildungsqualität, einer Lesetiefenänderung, einer Anzahl von Hintergrundrauschvorgängen und einer Anzahl von Proben, in denen die gleiche Variante unter Normalgewebe-Genomdaten detektiert wird.

9. Verfahren nach Anspruch 1, wobei das Extrahieren der Merkmale jedes Strukturvariantenkandidaten Folgendes umfasst:
Extrahieren, für jeden Strukturvariantenkandidaten, der Tumorhistologie, des Vollgenom-Duplikationsstatus, der Tumorreinheit in einer Probe und der Tumorploidie eines Tumorgenoms aus klinischen Daten.

10. Verfahren nach Anspruch 1, wobei das Machine-Learning-Modell Merkmale des Identifikationsziel-Strukturvariantenkandidaten empfängt und einen Wahrscheinlichkeitswert zum Klassifizieren des Identifikationsziel-Strukturvariantenkandidaten als positiv oder negativ ausgibt.

11. Verfahren nach Anspruch 1, das ferner Folgendes umfasst:
Bewerten der Klassifizierungsleistung des Machine-Learning-Modells unter Verwendung der Validierungsproben, die in den Ganzgenom-Sequenzierungsdaten enthalten sind; und
Bestimmen eines Grenzwerts zum Bestimmen, ob ein Wahrscheinlichkeitswert, der von dem Machine-Learning-Modell ausgegeben wird, positiv oder negativ anzeigt, basierend auf dem Klassifizierungs-Leistungsbewertungsergebnis des Machine-Learning-Modells.

12. Verfahren nach Anspruch 1, wobei das Erhalten von Strukturvariantenkandidaten, die aus den Ganzgenom-Sequenzierungsdaten identifiziert wurden, Folgendes umfasst:
Eingeben der Ganzgenom-Sequenzierungsdaten in ein Strukturvarianten-Suchwerkzeug; und
Erhalten von Strukturvariantenkandidaten, die durch das Strukturvarianten-Suchwerkzeug ausgegeben werden.

## Revendications

1. Procédé réalisé par un dispositif informatique pour l'identification de variantes structurales dans des génomes, comprenant :
l'obtention de variantes structurales candidates identifiées à partir de données de séquençage du génome entier, y compris une paire de données sur le génome tumoral et de données sur le génome des tissus normaux ;
l'extraction de caractéristiques de chaque variante structurale candidate d'après des données situées dans une plage prédéterminée autour de chaque variante structurale candidate dans les données de séquençage du génome entier ; et
l'étiquetage de chaque variante structurale candidate avec des informations de classification sur la base d'une liste de variantes structurales connues, chaque variante structurale candidate étant étiquetée comme positive si ladite variante structurale candidate existe dans la liste des variantes structurales connues, ladite variante structurale candidate étant étiquetée comme négative dans le cas contraire ; et
l'entraînement d'un modèle d'apprentissage automatique au moyen d'un ensemble de données, dans lequel sont stockées les caractéristiques extraites de chaque variante structurale candidate et les informations de classification étiquetées,
le modèle d'apprentissage automatique recevant une variante structurale candidate cible d'identification et délivrant une classification de la variante structurale candidate cible d'identification, le modèle d'apprentissage automatique recevant des caractéristiques de la variante structurale candidate cible d'identification et délivrant une valeur de probabilité pour classer la variante structurale candidate cible d'identification comme positive ou négative ;
**caractérisé en ce que**
l'extraction de caractéristiques de chaque variante structurale candidate comprend :
l'obtention de données d'une première longueur ou plus dans une direction dans laquelle des lectures d'appui de variante de ladite variante de structure candidate sont situées, à partir d'un point de cassure associé à ladite variante de structure candidate parmi les variantes de structure candidates dans les données de séquençage du génome entier, et
la première longueur étant une taille moyenne d'insert des données de séquençage du génome entier.

2. Procédé selon la revendication 1, comprenant en outre
la modification d'une position estimée d'une première variante structurale candidate sur la base d'informations d'étiquette d'alignement supplémentaire (SA) associées à la première variante structurale candidate parmi les variantes structurales candidates avant extraction des caractéristiques de chaque variante structurale candidate.

3. Procédé selon la revendication 1, comprenant en outre
la génération d'une liste de variantes structurales vraies par suppression de variantes structurales candidates identifiées comme variantes structurales négatives parmi les variantes structurales candidates obtenues.

4. Procédé selon la revendication 1, la modification de la position estimée de la première variante structurale candidate comprenant l'identification d'une première région et d'une seconde région sur une séquence de référence à laquelle le première variante structurale candidate candidat est associée,
la première région et la seconde région étant des régions non adjacentes l'une à l'autre.

5. Procédé selon la revendication 1, la modification de la position estimée de la première variante structurale candidate comprenant la détermination d'une position d'un point de cassure associé à la première variante structurale candidate.

6. Procédé selon la revendication 1, l'extraction de caractéristiques de chaque variante structurale candidate d'après des données situées dans une plage prédéterminée autour de chaque variante structurale candidate comprenant :
l'obtention de données d'une seconde longueur ou moins dans une direction opposée aux lectures d'appui de variante d'une première variante structurale candidate à partir d'un point de cassure associé à la première variante structurale candidate parmi les variantes structurales candidates dans les données de séquençage du génome entier.

7. Procédé selon la revendication 6, la seconde longueur étant de 200 paires de bases.

8. Procédé selon la revendication 1, l'extraction des caractéristiques de chaque variante structurale candidate comprenant l'extraction, pour chaque variante structurale candidate, d'un nombre de lectures d'appui de variante, d'un nombre de lectures fractionnées avec étiquette d'alignement supplémentaire, d'un nombre de lectures fractionnées, d'une qualité de mappage, d'un changement de profondeur de lecture, d'un nombre de lectures de bruit de fond et d'un nombre d'échantillons dans lesquels la même variante est détectée parmi les données de génome de tissu normal.

9. Procédé selon la revendication 1, l'extraction des caractéristiques de chaque variante structurale candidate comprenant l'extraction, pour chaque variante structurale candidate, de l'histologie tumorale, de l'état de duplication du génome entier, de la pureté tumorale dans un échantillon et de la ploïdie tumorale d'un génome tumoral à partir de données cliniques.

10. Procédé selon la revendication 1, le modèle d'apprentissage automatique recevant des caractéristiques de la variante structurale candidate cible d'identification et délivrant une valeur de probabilité pour classer la variante structurale candidate cible d'identification comme positive ou négative.

11. Procédé selon la revendication 1, comprenant en outre
l'évaluation des performances de classification du modèle d'apprentissage automatique en utilisant les échantillons de validation inclus dans les données de séquençage du génome entier ; et
la détermination d'une valeur de coupure pour déterminer si une valeur de probabilité délivrée à partir du modèle d'apprentissage automatique indique un caractère positif ou négatif sur la base du résultat d'évaluation de performances de classification du modèle d'apprentissage automatique.

12. Procédé selon la revendication 1, l'obtention de variantes structurales candidates identifiées à partir des données de séquençage du génome entier comprenant
l'introduction des données de séquençage du génome entier dans un outil de recherche de variantes structurales ; et
l'obtention de variantes structurales candidates délivrées par l'outil de recherche de variantes structurales.
